# EUROPÄISCHE PATENTSCHRIFT

(11) **EP 0 913 387 B1**
(45) Veröffentlichungstag und Bekanntmachung des Hinweises auf die Patenterteilung: **15.01.2003**
(21) Anmeldenummer: 98119852.6
(22) Anmeldetag: 20.10.1998
(51) Int. Cl.: C07C 209/48, C07C 209/52

(54) **Verfahren zur Herstellung von Isophorondiamin**
Process for the preparation of isophoronediamine
Procédé pour la préparation d'isophoronediamine

(30) Priorität: 30.10.1997 DE 19747913
(43) Veröffentlichungstag der Anmeldung: 06.05.1999
(73) Patentinhaber: Degussa AG, 40474 Düsseldorf (DE)
(72) Erfinder: Haas, Thomas Dr., 60316 Frankfurt (DE); Weber, Karl-Ludwig Dr., 64807 Dieburg (DE); Stadtmüller, Klaus, 63755 Alzenau (DE); Hofen, Willi, 63517 Rodenbach (DE); Vanheertum, Dr., 63796 Kahl (DE)

(56) Entgegenhaltungen:
- EP-A- 0 449 089
- EP-A- 0 659 734
- EP-A- 0 729 937
- DE-C- 19 540 191
- US-A- 3 773 832

## Beschreibung

Die Erfindung betrifft ein Verfahren zur Herstellung von Isophorondiamin (= 3-Aminomethyl-3,5,5-trimethylcyclohexylamin) durch aminierende Hydrierung von Isophoronnitril-imin (= 3-Cyano-3,5,5-trimethylcyclohexanimin) mit Wasserstoff in Gegenwart von Ammoniak und in Gegenwart eines Hydrierkatalysators auf der Basis eines oder mehrerer der Metalle Kobalt, Nickel und Ruthenium, und einer ein Hydroxylanion enthaltenden Base.

Durch katalytische Hydrierung von Aldiminen beziehungsweise Ketiminen in Gegenwart von Ammoniak, allgemein als aminierende Hydrierung bezeichnet, lassen sich in bekannter Weise primäre beziehungsweise sekundäre Amine herstellen. In analoger Weise sind durch aminierende Hydrierung von Nitrilen primäre Amine zugänglich. Geeignete Hydrierkatalysatoren sind insbesondere solche auf der Basis eines oder mehrerer der Metalle Kobalt, Nickel und Ruthenium, wobei die Katalysatoren zusätzlich herstellungsbedingt oder als Promotoren andere Metalle enthalten können. Es ist auch bekannt, die Selektivität der aminierenden Hydrierung durch die Anwesenheit eines Alkalioder Erdalkalihydroxids zu erhöhen.

Gemäß WO 92/21650 lassen sich Dinitrile mit hoher Selektivität in Aminonitrile überführen, indem die Hydrierung mit Wasserstoff unter Verwendung eines Raney-Nioder -Co-Katalysators in Gegenwart von Ammoniak und einer anorganischen Base, wie LiOH, NaOH oder KOH, durchgeführt wird.

Eine aminierende Hydrierung liegt auch der ein- oder zweistufigen Herstellung von Isophorondiamin aus Isophoronnitril (= 3-Cyano-3,5,5-trimethylcyclohexanon) zugrunde: Bei einstufiger Ausführung wird Isophoronnitril in Gegenwart von Ammoniak in situ iminiert und das entstandene Isophoronnitril-imin zu Isophorondiamin hydriert - siehe beispielsweise EP-A 0 659 734. Bei zweistufiger Ausführung wird in einer ersten Stufe in Anoder Abwesenheit eines sauren Iminierungskatalysators Isophoronnitril iminiert und das Isophoronnitril-imin enthaltende Reaktionsgemisch der Hydrierstufe zugeführt - siehe beispielsweise DE-PS 195 40 191, EP-B 0 449 089 und EP-A 0 729 937. Da es sich bei Isophorondiamin um ein großtechnisches Produkt handelt, richten sich die Bemühungen der Fachwelt darauf, ein möglichst reines Produkt in möglichst hoher Ausbeute zu gewinnen.

Die EP-A 0 449 089 lehrt ein zweistufiges Verfahren zur Herstellung von Isophorondiamin (IPDA) aus Isophoronnitril (IPN) bekannt, wobei in einer ersten Stufe IPN mit Ammoniak an aciden Metalloxiden zum Isophoronnitrilimin umgesetzt und dann das Imin in Gegenwart von Ammoniak und einem Hydrierkatalysator, insbesondere einem Katalysator auf der Basis von Kobalt und/oder Ruthenium, gegebenenfalls in Gegenwart von basischen Komponenten, wie Alkali- oder Erdalkalihydroxiden oder basischen Trägern, mit Wasserstoff zu IPDA hydriert wird. Bei der Nacharbeitung des Verfahrens gemäß EP-B 0 449 089, wobei jedoch als Iminierungskatalysator ein Sulfonsäuregruppen enthaltendes Organopolysiloxan gemäß DE-Patentanmeldung 196 27 265.3 eingesetzt und die Hydrierung bei 6 MPa anstelle 25 MPa durchgeführt wurde, zeigte sich, daß bei alleiniger Verwendung eines basischen Kobaltkatalysators (90 % Co, 5 % Mn und 1,9 % Na) die Ausbeute an Isophorondiamin mit zunehmender Betriebsdauer rasch abnimmt - siehe Vergleichsbeispiel 4. Es wurde ferner festgestellt - siehe Vergleichsbeispiele 5 bis 7 -, daß auch bei kontinuierlicher Zugabe eines Alkalihydroxids zur aminierenden Hydrierung von Isophoronnitril-imin, wie sie in der EP-A 0 729 937 empfohlen wird, in erheblichem Umfang noch Nebenprodukte entstehen, welche bei der destillativen Aufarbeitung des Reaktionsgemischs zu einer Minderung der Ausbeute an reinem Isophorondiamin führen.

Die Aufgabe der vorliegenden Erfindung besteht in der Bereitstellung eines weiter verbesserten Verfahrens zur Herstellung von Isophorondiamin aus Isophoronnitril-imin durch aminierende Hydrierung, wobei die Verbesserung in einer Minderung der Nebenproduktbildung und damit in einer Steigerung der Ausbeute bei gleichzeitig hoher Reinheit an aus dem Reaktionsgemisch der Hydrierstufe isolierbaren Aminen zu sehen ist.

Gelöst wird die Aufgabe durch ein Verfahren zur Herstellung von 3-Aminomethyl-3,5,5-trimethyl-cyclohexylamin (Isophorondiamin) aus 3-Cyano-3,5,5-trimethylcyclohexanimin (Isophoronnitril-imin), umfassend eine aminierende Hydrierung des Isophoronnitril-imins, mit Wasserstoff in Gegenwart von Ammoniak, einem Hydrierkatalysator auf der Basis von Kobalt, Nickel, Ruthenium oder Gemischen dieser Metalle und einer Hydroxid-Base bei einer Temperatur im Bereich von 50 bis 200 °C und einem Druck im Bereich von 0,3 bis 30 MPa und Aufarbeitung des Reaktionsgemischs, das dadurch gekennzeichnet ist, daß man die aminierende Hydrierung in Gegenwart eines quaternären Ammoniumhydroxids durchführt.

Wenngleich das Problem der Nebenproduktbildung und damit Ausbeuteminderung bei der aminierenden Hydrierung beliebiger Imine und Nitrile besteht, richten sich die nachfolgenden Ausführungen auf die Herstellung von Isophorondiamin aus Isophoronnitril-imin, wobei ein dieses Imin enthaltendes Reaktionsgemisch einer der aminierenden Hydrierung vorgeschalteten Iminierung von Isophoronnitril eingesetzt wird.

Bei den zu verwendenden quaternären Ammoniumhydroxiden handelt es sich um Basen der allgemeinen Formel (R¹R²R³R⁴N)⁺OH⁻, wobei R bis R⁴ gleich oder verschieden sein können und für aliphatische, cycloaliphatische oder aromatische Kohlenwasserstoffreste stehen. Vorzugsweise stehen R¹ bis R⁴ für aliphatische Kohlenwasserstoffreste, insbesondere solche mit 1 bis 12 C-Atomen, ganz besonders 1 bis 4 C-Atomen. Zweckmäßigerweise sind R¹, R² und R³ gleich und jeweils ein (C₁- bis C₄)-Alkyl und R⁴ (C₁- bis C₁₈)-Alkyl oder Aryl; Beispiele sind Phenyl- oder Lauryltrimethylammoniumhydroxid. Besonders bevorzugt werden Tetraalkylammoniumhydroxide, insbesondere solche aus der Reihe Tetramethyl-, Tetraethyl-, Tetra-n-propyl- und Tetran-butylammoniumhydroxid.

Zweckmäßigerweise erfolgt die Hydrierung in Gegenwart von 0,01 bis 100 mMol, insbesondere 0,05 bis 50 mMol und besonders bevorzugt 0,2 bis 20 mMol eines Tetraalkylammoniumhydroxids, jeweils bezogen auf ein Mol des Imins oder Nitrils.

Das erfindungsgemäße Verfahren kann batchweise oder kontinuierlich durchgeführt werden. Bei kontinuierlicher Fahrweise wird dem einen Suspensions- oder Festbett-Katalysator enthaltenden Reaktor außer dem Imin, Ammoniak und gegebenenfalls einem Lösungsmittel oder einem diese Stoffe enthaltenden Reaktionsgemisch aus einer vorgeschalteten Iminierungsstufe kontinuierlich Wasserstoff und zusätzlich ein quaternäres Ammoniumhydroxid zugeführt. Diese Base kann als Reinsubstanz oder als Lösung dem Reaktor oder gegebenenfalls bereits dem Reaktionsgemisch aus der Iminierungsstufe zugeführt werden. Besonders bevorzugt ist die Verwendung eines Festbettkatalysators, der in Rieselbettfahrweise betrieben wird.

Als Katalysatoren eignen sich insbesondere solche, deren hydrierwirksame Metalle ausgewählt sind aus der Reihe Kobalt, Nickel, Eisen und Ruthenium sowie anderen Edelmetallen oder Gemischen solcher Metalle. Bevorzugt verwendet werden Katalysatoren auf der Basis von Co, Ni und/oder Ru. Zusätzlich kann der Katalysator andere Metalle aus der Herstellung enthalten, etwa Aluminium, Zink oder Silicium, die bei der Herstellung von Raney-Katalysatoren in einer Raney-Legierung als auslaugbare Legierungskomponente anwesend waren. Zudem kann der Katalysator übliche Promotoren, beispielsweise solche aus der Reihe Cr, Fe, Co, Mn, Ta, Mo und Ti, enthalten.

Geeignet sind beispielsweise Raney-Katalysatoren, Vollkontakte oder trägergebundene Kontakte, etwa Co, Ni und/oder Ru auf Oxiden, wie Al₂O₃, TiO₂, ZrO₂, ZnO, MgO/Al₂O₃, wie sie in den zum Stand der Technik zitierten Dokumenten genannt sind. Besonders bevorzugt sind auch geformte Metall-Festbettkatalysatoren nach Raney, erhältlich nach dem in der DE-Patentanmeldung 197 21 897 beschriebenen Verfahren.

Die aminierende Hydrierung erfolgt in Anwesenheit von Ammoniak. Pro Mol Imin oder Nitril werden üblicherweise 2 oder mehr Mol NH₃, meistens 5 bis 500 Mol NH₃ eingesetzt. Ammoniak dient außer zur Aminierung teilweise oder ganz als Lösungsmittel.

Die Hydrierung kann in An- oder Abwesenheit hydrierstabiler organischer Lösungsmittel durchgeführt werden. Besonders zweckmäßig ist die Verwendung eines wasserlöslichen Alkohols, insbesondere eines niederen Alkohols mit 1 bis 4 C-Atomen, vorzugsweise Methanol, oder eines wasserlöslichen Ethers, wie Tetrahydrofuran. Auch Glykole sind als Lösungsmittel geeignet, sofern ihr Siedepunkt ausreichend weit von denjenigen der Reaktionsprodukte der aminierenden Hydrierung liegt.

Die aminierende Hydrierung wird zweckmäßigerweise bei niedrigem bis mäßigem Druck, etwa 0,3 bis 10 MPa, kann aber auch bei hohem Druck, also im Bereich von über 10 bis 30 MPa, erfolgen. Die Temperatur während der Hydrierung liegt zweckmäßigerweise im Bereich von etwa 50 bis 250 °C, insbesondere 50 bis 150 °C, sie kann jedoch auch außerhalb der genannten Grenzen liegen. In vielen Fällen kann es zweckmäßig sein, die Temperatur während der Hydrierung kontinuierlich oder stufenweise zu erhöhen. Wasserstoff kann bei kontinuierlicher Betriebsweise im Gleichstrom oder Gegenstrom zum Imin enthaltenden Reaktionsgemisch geführt werden. Die Hydrierung kann in einem einzigen Reaktor oder in mehreren hintereinander geschalteten Reaktoren in Rieselbett- oder Sumpffahrweise durchgeführt werden.

Bei der Herstellung von Isophorondiamin wird vorzugsweise ein Isophoronnitril-imin enthaltendes Reaktionsgemisch der aminierenden Hydrierung zugeführt. Das genannte Gemisch kann unmittelbar jenes sein, wie es bei der Iminierung von Isophoronnitril mit Ammoniak in Gegenwart oder Abwesenheit eines organischen Lösungsmittels, wie insbesondere Methanol, in An- oder Abwesenheit eines sauren Iminierungskatalysators erhalten wird oder wie es aus einem derartigen Reaktionsgemisch nach Zugabe oder Abdestillieren von Lösungsmitteln und/oder eines Teils des Ammoniaks erhältlich ist. Die genannte Iminierung wird vorzugsweise in Gegenwart eines Iminierungskatalysators, wie eines aciden Metalloxids, eines sauren Zeoliths oder eines sauren Ionenaustauschers, etwa eines Sulfonatgruppen enthaltenden Organopolysiloxans gemäß DE-Patentanmeldung 196 27 265.3, durchgeführt. Durch die Anwesenheit eines niederen Alkohols, wie insbesondere Methanol, ist es möglich, bei tieferem Druck aminierend zu hydrieren als in Abwesenheit des genannten Alkohols.

Wie aus den nachfolgend beschriebenen Beispielen und Vergleichsbeispielen folgt, gelingt es in überraschender Weise, die Ausbeute an reinem Isophorondiamin dadurch um einige Prozentpunkte zu steigern, daß anstelle eines Alkali- oder Erdalkalihydroxids ein quaternäres Ammoniumhydroxid eingesetzt wird.

### Beispiele

Hergestellt wurde Isophorondiamin durch Iminierung von Isophoronnitril in Gegenwart eines Iminierungskatalysators und Methanol als Lösungsmittel (= erste Stufe) und aminierende Hydrierung des Reaktionsgemischs aus der ersten Stufe (= zweite Stufe). Das Reaktionsgemisch der zweiten Stufe wurde bezüglich der Zusammensetzung gaschromatographisch analysiert und destillativ aufgearbeitet.

In der Iminierung aller Beispiele und Vergleichsbeispiele wurde ein Sulfonatgruppen enthaltendes Organopolysiloxan als Iminierungskatalysator eingesetzt (siehe DE 196 27 265.3). Der Katalysator war als Festbett in einem Reaktorrohr (Innendurchmesser 20 mm, Länge 250 mm, Katalysator-Befüllung 30 ml) angeordnet. Der Iminierungsreaktor wurde bei 35 °C in Sumpf fahrweise betrieben. Die Einsatzlösung bestand aus 15 Gew.-% Isophoronnitril, 30 Gew.-% Ammoniak und 55 Gew.-% Methanol.

Diese Lösung wurde mit einem Massenstrom von 80 ml/h von unten nach oben durch den Iminierungsreaktor geleitet.

Das Reaktionsgemisch der ersten Stufe wurde von oben auf den mit einem Festbettkatalysator bestückten Hydrierreaktor (Innendurchmessser 17 mm, Länge 350 mm, Befüllung 150 ml Katalysator) gegeben (Rieselbettfahrweise). Die Base wurde unmittelbar vor dem Hydrierreaktor in das Reaktionsgemisch gegeben. Der Wasserstoff wurde mit einem Volumenstrom von 36 l/h von oben in den Reaktor eingeleitet. Die Hydrierung erfolgte bei 100 °C und einem Druck bei 6 MPa. Das Produktgemisch aus dem zweiten Reaktor wurde in einer Vorlage aufgefangen und in üblicher Weise aufgearbeitet. Als Hydrierkatalysatoren wurden eingesetzt:
- K1:: Geformter Co-Festbettkatalysator nach Raney, hergestellt und aktiviert gemäß Beispiel 1 von DE 197 21 897.0.
- K2:: Geformter Co-Festbettkatalysator gemäß EP-A 0 648 534 - siehe auch Vergleichsbeispiel 1 in der vorgenannten DE 197 21 897.0.
- K3:: Handelsüblicher Kobalt-Trägerkatalysator (Co auf einem Silikat).
- K4:: Reduzierter Co-Katalysator mit 90 % Co, 5 % Mn und 1,9 % Na im Form von 4 mm-Strängen (Katalysator analog zu dem im Verfahren der EP-A 0 449 089 eingesetzten Katalysator).

Details zu K1, K2 und K3 sind der Tabelle 1 zu entnehmen.

**Tabelle 1**

| Abmessungen | K1 | K2 | K3 |
|---|---|---|---|
| | 5 ⌀ x 5 | 5 ⌀ x 5 | 4,5 ⌀ x 5 |
| Kobalt (Gew.-%) | 60 | 60 | 45 |
| Aluminium (Gew.-%) | 40 | 40 | - |
| Schüttdichte (kg/l) | 1,2 | 2,2 | 0,74 |
| Porenvolumen (cm³/g) | 0,3 | 0,05 | 0,3 |
| Schalendicke (mm) | 0,8 | 0,3 | n.a. |
| Bruchfestigkeit (N) | 120 | 300 | 80 |

Als Base wurden NaOH oder Tetramethylammoniumhydroxid (TMAH) eingesetzt und zwar jeweils in einer Menge von 5 mMol Base pro Mol des in der ersten Stufe eingesetzten Isophoronnitrils. In den Vergleichsversuchen 1 bis 4 wurde keine Base eingesetzt.

Tabelle 2 enthält Angaben zur Ausbeute an Isophorondiamin (gaschromatographisch bestimmt) sowie zur Reinheit des aus der Destillation des Reaktionsgemischs erhaltenen Isophorondiamins der Beispiele (B1 bis B3) und Vergleichsbeispiele VB1 bis VB7.

**Tabelle 2**

| Nr. | Katalysator | Base | Ausbeute | Reinheit |
|---|---|---|---|---|
| VB 1 | K 1 | - | 89,7 | 99,9 |
| VB 2 | K 2 | - | 89,1 | 99,75 |
| VB 3 | K 3 | - | 84,3 | 99,85 |
| VB 4 | K 4 | Na₂O im Katalysator | 87,6 *) | |
| | | | 84,5 **) | |
| B 1 | K 1 | TMAH | 95,7 | 99,9 |
| VB 1 | K 1 | NaOH | 92,4 | 99,9 |
| B 2 | K 2 | TMAH | 95 | 99,75 |
| VB 2 | K 2 | NaOH | 92,1 | 99,75 |
| B 3 | K 3 | TMAH | 90,2 | 99,75 |
| VB 3 | K 3 | NaOH | 87,1 | 99,7 |

| | | | | |
|---|---|---|---|---|
| *) nach 21 h Betrieb | | | | |
| **) nach 91 h Betrieb | | | | |

Die Versuche zeigen die Ausbeutesteigerung, wenn NaOH gegen TMAH ausgetauscht wird. Der Effekt tritt mit unterschiedlichen Katalysatoren auf. Ein Na enthaltender Katalysator (K4) weist eine ungenügende Standzeit auf.

## Patentansprüche

1. Verfahren zur Herstellung von 3-Aminomethyl-3,5,5-trimethylcyclohexylamin (Isophorondiamin) aus 3-Cyano-3,5,5-trimethylcyclohexanimin (Isophoronnitril-imin), umfassend eine aminierende Hydrierung des Isophoronnitril-imins, mit Wasserstoff in Gegenwart von Ammoniak, einem Hydrierkatalysator auf der Basis von Kobalt, Nickel, Ruthenium oder Gemischen dieser Metalle und einer Hydroxid-Base bei einer Temperatur im Bereich von 50 bis 250 °C und einem Druck im Bereich von 0,3 bis 30 MPa und Aufarbeitung des Reaktionsgemischs,
**dadurch gekennzeichnet,**
**daß** man die aminierende Hydrierung in Gegenwart eines quaternären Ammoniumhydroxids durchführt.

2. Verfahren nach Anspruch 1,
**dadurch gekennzeichnet,**
**daß** man als quaternäres Ammoniumhydroxid Tetramethyl-, Tetraethyl-, Tetra-n-propyl- oder Tetra-nbutylammoniumhydroxid einsetzt.

3. Verfahren nach Anspruch 1 oder 2,
**dadurch gekennzeichnet,**
**daß** man die aminierende Hydrierung in Gegenwart von 0,05 bis 50 mMol Tetraalkylammoniumhydroxid pro Mol Isophoronnitril-imin durchführt.

4. Verfahren zur Herstellung von Isophorondiamin nach einem der Ansprüche 1 bis 3,
**dadurch gekennzeichnet,**
**daß** man Isophoronnitril-imin in Gegenwart von 0,2 bis 20 mMol eines quaternären Ammoniumhydroxids, insbesondere eines Tetra(C₁- bis C₄-)alkylammoniumhydroxids, pro Mol Isophoronnitril-imin in An- oder Abwesenheit eines organischen Lösungsmittels bei einer Temperatur im Bereich von 50 bis 150 °C ein- oder mehrstufig aminierend hydriert.

5. Verfahren nach Anspruch 4,
**dadurch gekennzeichnet,**
**daß** man die aminierende Hydrierung in einem Festbettoder Suspensions-Reaktor durchführt, wobei man dem Reaktor kontinuierlich ein Isophoronnitril-imin enthaltendes Reaktionsgemisch, erhalten durch Iminierung von Isophoronnitril in Gegenwart eines Überschusses an Ammoniak in An- oder Abwesenheit eines sauren Iminierungskatalysators und in An- oder Abwesenheit eines organischen Lösungsmittels, und ein quaternäres Ammoniumhydroxid zuführt und das den Reaktor verlassende Reaktionsgemisch destillativ aufarbeitet.

6. Verfahren nach Anspruch 4 oder 5,
**dadurch gekennzeichnet,**
**daß** man die aminierende Hydrierung in einem Festbettreaktor in Rieselbettfahrweise durchführt, wobei das aminierend zu hydrierende Reaktionsgemisch im Reaktor eine oder mit steigender Temperatur angerordnete mehrere Temperaturstufen durchläuft.

## Claims

1. A process for preparing 3-aminomethyl-3,5,5-trimethylcyclohexylamine (isophoronediamine) from 3-cyano-3,5,5-trimethylcyclohexanimine (isophoronenitrilimine), comprising an aminative hydrogenation of isophoronenitrilimine by means of hydrogen in the presence of ammonia, a hydrogenation catalyst based on cobalt, nickel, ruthenium or a mixture of these metals and a hydroxide base at a temperature in the range from 50 to 250°C and a pressure in the range from 0.3 to 30 MPa and work-up of the reaction mixture, **characterized in that** the aminative hydrogenation is carried out in the presence of a quaternary ammonium hydroxide.

2. A process according to claim 1, **characterized in that** tetramethylammonium, tetraethylammonium, tetra-n-propylammonium or tetra-n-butylammonium hydroxide is used as quaternary ammonium hydroxide.

3. A process according to claim 1 or 2, **characterized in that** the aminative hydrogenation is carried out in the presence of from 0.05 to 50 mmol of tetraalkylammonium hydroxide per mole of isophoronenitrilimine.

4. A process for preparing isophoronediamine according to any one of claims 1 to 3, **characterized in that** isophoronenitrilimine is aminatively hydrogenated in one or more stages at a temperature in the range from 50 to 150°C in the presence of from 0.2 to 20 mmol of a quaternary ammonium hydroxide, in particular a tetra(C₁-C₄-)alkylammonium hydroxide, per mole of isophoronenitrilimine and in the presence or absence of an organic solvent.

5. A process according to claim 4, **characterized in that** the aminative hydrogenation is carried out in a fixed-bed or suspension reactor, with an isophoronenitrilimine-containing reaction mixture obtained by imination of isophoronenitrile in the presence of an excess of ammonia in the presence or absence of an acid imination catalyst and in the presence or absence of an organic solvent, and a quaternary ammonium hydroxide being fed continuously into the reactor and the reaction mixture leaving the reactor being worked up by distillation.

6. A process according to claim 4 or 5, **characterized in that** the aminative hydrogenation is carried out in a fixed-bed reactor operated in the downflow mode, with the reaction mixture to be hydrogenated aminatively passing through one or more temperature stages arranged in order of increasing temperature in the reactor.

## Revendications

1. Procédé de préparation de 3-aminométhyl-3,5,5-triméthylcyclohexylamine (isophorone diamine) à partir de la 3-cyano-3,5,5-triméthylcyclohexanimine (isophorone nitrile-imine) comprenant une hydrogénation aminante de l'isophorone nitrile-imine avec de l'hydrogène en présence d'ammoniac, d'un catalyseur d'hydrogénation à base de cobalt, de nickel, de ruthénium ou de mélanges de ces métaux, et d'une base d'hydroxyde à une température dans la zone allant de 50 à 250°C et sous une pression dans la zone allant de 0,3 à 30 Mpa et traitement du mélange réactionnel,
**caractérisé en ce qu'**
on effectue l'hydrogénation aminante en présence d'un hydroxyde d'ammonium quaternaire.

2. Procédé selon la revendication 1,
**caractérisé en ce qu'**
on met en oeuvre comme hydroxyde d'ammonium quaternaire, l'hydroxyde de tétraméthylammonium, l'hydroxyde de tétraéthylammonium, l'hydroxyde de tétra n-propylammonium, ou l'hydroxyde de tétra n-butylammonium.

3. Procédé selon l'une quelconque des revendications 1 ou 2,
**caractérisé en ce qu'**
on effectue l'hydrogénation aminante en présence de 0,05 à 50 mmol d'hydroxyde de tétraalkylammonium par Mol d'isophoronenitrileimine.

4. Procédé de préparation d'isophorone diamine selon l'une des revendications 1 à 3,
**caractérisé en ce qu'**
on hydrogènise d'une manière aminante l'isophorone nitrile-imine en présence de 0,2 à 20 mmol d'un hydroxyde d'ammonium quaternaire par mole d'isophorone nitrile-imine, en présence ou en l'absence d'un solvant organique à une température dans al plage de 50 à 150°C, en une ou plusieurs étapes.

5. Procédé selon la revendication 4,
**caractérisée en ce qu'**
on effectue l'hydrogénation aminante dans un réacteur à lit fixe ou en suspension dans lequel on amène au réacteur en continu un mélange réactionnel contenant une isophoronenitrile-imine, obtenue par imination de l'isophoronenitrile en présence d'un excès d'ammoniac, en présence ou un l'absence d'un catalyseur acide d'imination et en présence ou en l'absence d'un solvant organique, et un hydroxyde d'ammonium quaternaire, et on traite par distillation le mélange réactionnel qui sort du réacteur.

6. Procédé selon la revendication 4 ou la revendication 5,
**caractérisé en ce qu'**
on effectue l'hydrogénation aminante dans un réacteur à lit fixe dans un mode opératoire en lit de ruissellement, dans lequel le mélange réactionnel aminant à hydrogéner traverse dans le réacteur un ou plusieurs stades de température disposés avec une température croissante.
